# EUROPÄISCHE PATENTSCHRIFT

(11) **EP 1 112 095 B1**
(45) Veröffentlichungstag und Bekanntmachung des Hinweises auf die Patenterteilung: **20.11.2002**
(21) Anmeldenummer: 99946158.5
(22) Anmeldetag: 10.09.1999
(51) Int. Cl.: A61L 27/34, A61L 27/54, A61L 31/08, A61L 31/16

(54) **BIOLOGISCH AKTIVE IMPLANTATE**
BIOLOGICALLY ACTIVE IMPLANTS
IMPLANTS AYANT UNE ACTIVITE BIOLOGIQUE

(30) Priorität: 11.09.1998 DE 19843251
(43) Veröffentlichungstag der Anmeldung: 04.07.2001
(73) Patentinhaber: Schmidmaier, Gerhard, Dr., 10717 Berlin (DE); Raschke, Michael, Dr., 12205 Berlin (DE); Stemberger, Axel, Dr., D-85579 Neubiberg (DE)
(72) Erfinder: SCHMIDMAIER, Gerhard, Dr., D-10717 Berlin (DE); RASCHKE, Michael, D-12205 Berlin (DE); STEMBERGER, Axel, D-85579 Neubiberg (DE)
(74) Vertreter: Glawe, Delfs, Moll & Partner
(86) Internationale Anmeldenummer: EP9906708
(87) Internationale Veröffentlichungsnummer: WO00015273

(56) Entgegenhaltungen:
- EP-A- 0 652 017
- WO-A-93/20859
- WO-A-97/47254

## Beschreibung

Die Erfindung betrifft ein Implantat zur Versorgung von pathologischen Veränderungen am Stütz- und/oder Bewegungsapparat. Gegenstand der Erfindung ist ferner ein Verfahren zur Herstellung eines solchen Implantats. Implantate der genannten Art sind bekannt. Sie sollen bspw. eine Fraktur mechanisch stabilisieren und so den Heilungsprozeß fördern oder im Falle von Endoprothesen dauerhaft mit dem Knochen verbunden sein.

Es ist bspw. aus WO-A 9819699 bekannt, zur Förderung der Osteosynthese und damit Beschleunigung der Frakturheilung Medikamente oder Hormone systemisch zu verabreichen. Bspw. können zu diesem Zweck Wachstumsfaktoren wie IGF-I verwendet werden. Diese systemische Applikation kann zu unerwünschten Nebenwirkungen führen.

WO-A 9320859 offenbart die Herstellung einer dünnen Folie bzw. eines Films aus einem Polymilchsäure/Polyglykolsäurecopolymer, in die Wachstumsfaktoren eingearbeitet sind. Mit einem solchen Film sollen beispielsweise Frakturfixationsvorrichtungen vor deren Verwendung umwickelt werden. Die Wachstumsfaktoren sollen so lokal im Bereich der Fraktur freigesetzt werden. In der Praxis ist dieses Verfahren nicht anwendbar, da sich bspw. ein mit einem entsprechenden Film umwickelter Nagel nicht in einer Art und Weise intramedullär einbringen läßt, daß der den Nagel lediglich lose umhüllende Film tatsächlich an den vorgesehenen Wirkort gelangt.

EP-A-0 652 017 offenbart die Beschichtung von Biomaterialien wie Infusionskathetern, Nahtmaterial oder Stents mit einem lackartigen Überzug. Die Schrift befaßt sich nicht mit Implantaten zur Versorgung von pathologischen Veränderungen am Stütz- und/oder Bewegungsapparate.

Der vorliegenden Erfindung liegt daher die Aufgabe zugrunde, ein Implantat der eingangs genannten Art zu schaffen, das eine heilungsfördernde Wirkung auf pathologische Veränderungen am Stütz- und Bewegungsapparate, insbesondere eine die Osteosynthese fördernde und damit die Frakturheilung bzw. das Einwachsen eines Implantats beschleunigende Wirkung aufweist.

Die Lösung gemäß der Erfindung besteht bei einem solchen Implantat darin, daß es eine lackartige Beschichtung einer Stärke von 100 µm oder weniger aus einem biologisch abbaubaren Polymer aufweist, wobei die lackartige Beschichtung erhältlich ist durch ein Verfahren mit den Schritten:
- Herstellen einer Dispersion des biologisch abbaubaren Polymers in einem organischen Lösungsmittel,
- Aufbringen der Dispersion auf die zu beschichtende Oberfläche,
- Abdampfenlassen des Lösungsmittels in einer mit Lösungsmitteldampf im wesentlichen gesättigten Gasatmosphäre.

Zunächst seien einige im Rahmen der Erfindung verwendete Begriffe erläutert. Der Begriff Implantat bezeichnet eine Vorrichtung, die bei der Anwendung durch einen chirurgischen Eingriff wenigstens teilweise ins Körperinnere eingebracht wird. Ein solches Implantat dient der Versorgung von pathologischen Veränderungen am Stütz- und/oder Bewegungsapparat insbesondere durch mechanische Einwirkung. Solche pathologischen Veränderungen können bspw. Frakturen, pathologische Veränderungen von Gelenken und Knochen, Dehnungen oder Risse von Bändern oder Sehnen oder dergleichen sein. Gemeinsam ist den erfindungsgemäßen Implantaten, daß sie bei der Anwendung in unmittelbarem Kontakt mit einem Knochen oder einem anderen Element des Stütz- und Bewegungsapparats (bspw. Bändern, Sehnen) geraten bzw. zur Befestigung an oder in einem Knochen oder sonstigem Element vorgesehen sind.

Der Begriff Frakturfixationsvorrichtung bezeichnet jegliche Vorrichtung, die dem Fixieren, dem Korrigieren und/oder der mechanischen Stabilisierung einer Knochenfraktur dient. Beispielhaft seien Platten, Schrauben, Nägel, Stifte, Drähte, Fäden oder Cages für den Stütz- und Bewegungsapparat genannt. In der Regel werden solche Frakturfixationsvorrichtungen nach Ausheilung der Fraktur wieder entfernt, unter Umständen können diese jedoch auch dauerhaft im oder am Knochen verbleiben, oder vom Organismus resorbiert werden.

Endoprothesen sind zum dauerhaften Verbleib im Körper bestimmt und ersetzen in der Regel die Funktion eines natürlichen Organs wie bspw. eines Gelenks, Knochenteils oder Zahns.

Der Begriff des Implantats ist im weitesten Sinne zu verstehen und schließt bspw. auch solche Implantate ein, die für Verlängerungs- und Verkürzungsosteotomien, bei Craniotomien, für die Bandheilung und -remodellierung, bei der Tumor- und Sportchirurgie, im Dentalbereich sowie bei Distraktionen im Mund-, Kiefer- und Gesichtsbereich Verwendung finden.

Die Implantate sind aus einem Grundmaterial gefertigt, das sich von dem Material der lackartigen Beschichtung chemisch und/oder physikalisch unterscheidet. Häufig wird es sich bei dem Grundmaterial um ein nicht bioabbaubares Material handeln. Dies bedeutet, daß das Material unter den am Einsatzort im Körper herrschenden Bedingungen und während eines üblichen Verweilzeitraums im Körper nicht oder allenfalls in einer die gewünschte Wirkung nicht oder nur unwesentlich beeinträchtigender Weise abgebaut, angegriffen oder sonstwie verändert wird. Häufig wird ein erfindungsgemäßes Implantat aus einem Metall oder einer Metallegierung bestehen, bspw. einem Edelstahl oder Titan. Alternativ kann das Implantat aus einem Grundmaterial bestehen, das selbst bioabbaubar bzw. bioresorbierbar ist, das jedoch ohne die erfindungsgemäße lackartige Beschichtung die unten geschilderten vorteilhaften Eigenschaften nicht aufweist.

Erfindungsgemäß weisen die Implantate eine lackartige Beschichtung auf. Lackartig bedeutet, daß die Beschichtung mit der Oberfläche des Grundmaterials eine haftende Verbindung einer solchen Festigkeit eingeht, daß die Beschichtung während der Applikation des Implantats durch mechanische Einwirkung nicht oder nur in einem solchen Maße abgerieben oder sonstwie beschädigt wird, daß der unten noch näher geschilderte technische Effekt nicht beeinträchtigt wird. Bspw. muß ein Knochennagel mit einer lackartigen Beschichtung in vorgesehener Weise eingeschlagen werden können, ohne daß es zu einem wesentlichen Abrieb der lackartigen Beschichtung kommt.

Die Beschichtung besteht aus einem biologisch abbaubaren Polymer. Dies bedeutet, daß sie unter den an der Implantationsstelle herrschenden physiologischen Bedingungen nach und nach in einem Zeitraum von vorzugsweise mehreren Wochen oder Monaten in molekulare Spaltprodukte abgebaut wird. Diese Spaltprodukte und etwaige weitere Metaboliten weisen vorzugsweise keine oder allenfalls eine geringe Toxizität auf und können vorzugsweise vollständig oder weitgehend vom Körper metabolisiert oder ausgeschieden werden. Man bezeichnet solche ohne toxische Metaboliten vollständig abbaubare und ausscheidbare Polymere auch als bioresorbierbar. Bevorzugt sind die erfindungsgemäß verwendeten Polymere bioresorbierbar.

Die Erfindung beruht auf der überraschenden Erkenntnis, daß bereits eine erfindungsgemäße lackartige Beschichtung ohne den Zusatz weiterer pharmazeutischer Wirkstoffe wie bspw. Wachstumsfaktoren sowohl eine osteosynthetische und damit die Frakturheilung fördernde als auch eine anti-infektiöse und damit Komplikationen vermeidende Wirkung aufweist.

Die lackartige Beschichtung weist vorzugsweise eine Stärke von 50 µm, weiter vorzugsweise etwa 30 µm oder weniger, weiter vorzugsweise etwa 20 µm oder weniger auf. Häufig ist eine Stärke von 10 bis 30 µm, vorzugsweise 10 bis 20 µm bevorzugt.

Vorzugsweise besitzt das verwendete Polymer eine Glasübergangstemperatur von 37°C oder höher, damit es im Körper die gewünschte Festigkeit behält. Polymere mit einem mittleren Molekulargewicht von 100 kDa oder weniger sind im Rahmen der Erfindung bevorzugt.

Bevorzugt ist das Polymer ausgewählt aus der Gruppe bestehend aus Poly-α-Hydroxysäuren, Polyglykolen, Polytyrosincarbonaten, Stärke, Gelatine, Cellulose sowie Mischungen und Mischpolymerisaten aus diesen Bestandteilen. Unter den Poly-α-Hydroxysäuren sind besonders bevorzugt Polylaktide, Polyglykolsäuren und Mischpolymerisate daraus. Ein geeignetes Polylaktid ist bspw. unter Bezeichnung Resomer R 203 von Boehringer-Ingelheim erhältlich. Es handelt sich um ein racemisches Poly-D,L-Laktid. Dieses Racemat bildet eine amorphe lackartige Schicht auf der Implantatoberfläche. Die Bildung kristalliner Polymerstrukturen in der Beschichtung sollte vorzugsweise vermieden werden, daher wird man in der Regel kein enantiomerenreines Laktid verwenden. Unter den Polytyrosincarbonaten eignen sich bspw. p(DTE-co-5% PEG 1000 carbonate) und p(DTE-co-26% PEG 20000 carbonate). Es handelt sich hierbei um Copolymere, die den angegebenen Anteil Polyethylenglykole enthalten.

Im Rahmen der Erfindung kann die Beschichtung zusätzliche pharmazeutisch wirksame Stoffe, bspw. osteoinduktive oder biozide bzw. antiinfektiöse Materialien enthalten. Als osteoinduktive Materialien kommen bspw. Wachstumsfaktoren in Frage, deren Anteil am Gesamtgewicht der Beschichtung vorzugsweise 0,1 bis 10 Gew.-%, weiter vorzugsweise 0,5 bis 8 Gew.-%, besonders bevorzugt 1 bis 5 Gew.-% beträgt. Dieser Gewichtsanteil bezieht sich auf die reine Wirksubstanz ohne etwaige pharmazeutische Trägerstoffe.

Die Wachstumsfaktoren können ausgewählt sein aus der Gruppe der IGF (insulin like growth factors), TGF (transforming growth factors), FGF (fibroblast growth factors), EGF (epidermal growth factors), BMP (bone morphogenic proteins) sowie der PDGF (platelet derived growth factors). Diese Wachstumsfaktoren sind dem Fachmann geläufig und kommerziell erhältlich.

Bevorzugt enthält die lackartige Beschichtung die Wachstumsfaktoren IGF-I oder TGF-β, besonders bevorzugt ist eine Kombination dieser beiden Wachstumsfaktoren.

Gegenstand der Erfindung ist ferner ein Verfahren zur Herstellung eines vorstehend beschriebenen Implantats mit den Schritten:
- Herstellen einer Dispersion des biologisch abbaubaren Polymers in einem organischem Lösungsmittel,
- Aufbringen der Dispersion auf die zu beschichtende Oberfläche,
- Abdampfenlassen des Lösungsmittels in einer mit Lösungsmitteldampf im wesentlichen gesättigten Gasatmosphäre.

Der Begriff Dispersion bezeichnet dabei jedwede Verteilung des Polymers in einem organischen Lösungsmittel. Es kann sich um eine Lösung im chemischen Sinne, um eine rein physikalische Dispersion sowie um Zwischenstufen wie insbesondere kolloidale Lösungen handeln.

Das Aufbringen der Dispersion und das Abdampfenlassen des Lösungsmittels erfolgt bevorzugt bei einer Temperatur von 0 bis 30°C, weiter vorzugsweise bei Raumtemperatur von etwa 22°C. Durch diese sogenannte kalte Beschichtung lassen sich auch temperaturempfindliche Bestandteile wie bspw. bestimmte Wachstumsfaktoren zusammen mit dem Polymer auf das Implantat aufbringen. Das Aufbringen geschieht vorzugsweise durch Eintauchen des Implantat in die Dispersion. Andere Arten des Aufbringens wie Bestreichen, Aufsprühen etc. sind ebenfalls denkbar. Es versteht sich, daß die Dispersion zusätzlich zu dem Polymer ggf. die genannten weiteren pharmazeutischen Wirkstoffe wie osteoinduktive oder biozide Materialien enthalten kann.

Das Abdampfenlassen des Lösungsmittels erfolgt in einer mit Lösungsmitteldampf im wesentlichen gesättigten Gasatmosphäre. Zu diesem Zweck wird vorzugsweise das in die Dispersion eingetauchte Implantat in einem abgeschlossenen Raum, in dessen Atmosphäre eine hohe Lösungsmittelsättigung herrscht, bewegt. Man erreicht so ein sehr langsames Abdampfen des Lösungsmittels, so daß eine gleichförmige, feste lackartige Beschichtung entsteht. Bevorzugt dauert das Abdampfen 1 min bis 1 h, weiter vorzugsweise 5 - 30 min, besonders bevorzugt etwa 10 min.

Bevorzugt ist es ferner, die Beschichtung aus mehreren dünnen Schichten aufzubauen und somit das Aufbringen der Dispersion und Abdampfenlassen des Lösungsmittels zweimal oder ggf. mehrmals zu wiederholen

Besonders bevorzugt wird im Rahmen der Erfindung eine Dispersion verwendet, die eine kolloidale Lösung des Polymers im Lösungsmittel ist. Bevorzugt sind in dieser kolloidalen Lösung polymere Kolloidteilchen enthalten, deren Größe zwischen 1 und 1000 nm liegt und bevorzugt unter 400 bis 500 nm liegen sollte. Bspw. kann man eine solche kolloidale Lösung herstellen, in dem man Polymer und Lösungsmittel miteinander mischt und für einen Zeitraum von 1 min bis 24 h, vorzugsweise 2 bis 24 h, weiter vorzugsweise 3 bis 12 h, weiter vorzugsweise 4 bis 8 h, besonders bevorzugt etwa 6 h stehen läßt. Innerhalb des besonders bevorzugten Zeitraums von etwa 6 h bilden sich polymere Kolloidteilchen der gewünschten Größenordnung unterhalb von etwa 500 nm.

Zum Abtrennen von etwaig verbleibenden größeren Polymerpartikeln der Dispersion kann man die kolloidale Lösung vor dem Aufbringen auf das Implantat filtrieren, bevorzugt ist dabei ein Mikroporenfilter, dessen Porengröße der gewünschten maximalen Kolloidteilchengröße entspricht und 0,45 µm oder kleiner ist. Käuflich erhältlich sind bspw. Mikroporenfilter mit einer Porengröße von 0,45 oder 0,2 µm.

Als Lösungsmittel werden bevorzugt gängige organische nicht oder schwach polare Lösungsmittel benutzt. Besonders bevorzugt werden im Rahmen der Erfindung Ethylacetat oder Chloroform verwendet.

Die Dispersion enthält vor dem Aufbringen auf das Implantat vorzugsweise 20 bis 300, weiter vorzugsweise 50 bis 150 mg Polymer (ggf. einschließlich weiterer Inhaltsstoffe wie osteoinduktiver oder biozider Materialien) pro ml Lösungsmittel.

Ausführungsbeispiele der Erfindung werden im folgenden anhand der Zeichnung erläutert. Darin zeigen:
- Fig. 1: den biologischen Abbau einer Polylaktidbeschichtung auf einem erfindungsgemäßen Implantat im zeitlichen Verlauf in vivo und in vitro;
- Fig. 2: die Freisetzung der in die Polylaktidbeschichtung eingearbeiteten Wachstumsfaktoren im zeitlichen Verlauf;
- Fig. 3: einen radiologischen Vergleich der Wirkung erfindungsgemäßer gegenüber unbehandelten (unbeschichteten) Implantaten auf die Frakturheilung bei Ratten;
- Fig. 4: einen biomechanischen Vergleich dieser Implantate;
- Fig. 5: einen histomorphometrischen Vergleich dieser Implantate;
- Fig. 6: Bilder der histomorphometrischen Untersuchungen;
- Fig. 7: radiologischer Vergleich beschichteter und unbeschichteter Implantate bei Yukatan-Schweinen;

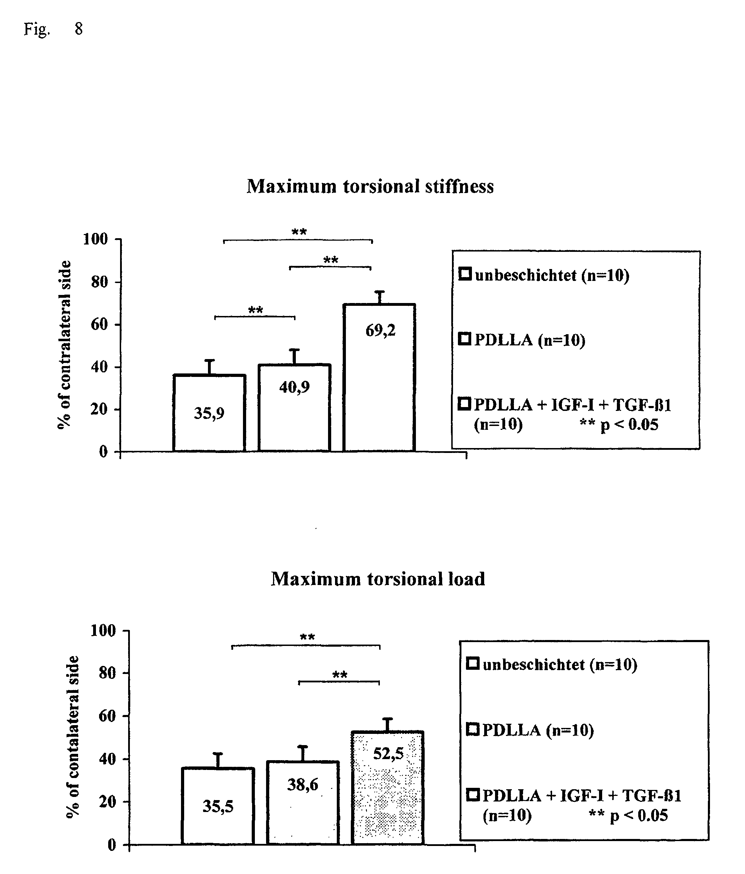

- Fig. 8: biomechanischer Vergleich der entsprechenden Implantate der Fig. 7;
- Fig. 9: maximales Drehmoment und torsionale Steifigkeit einer weiteren Tibia-Fraktur-Untersuchung bei Ratten.

### Beispiel 1: Herstellung eines erfindungsgemäßen Implantats

400 mg PDLLA (Poly(D,L)-laktid, Resomer R 203, Boehringer-Ingelheim) werden in 6 ml Chloroform bei Raumtemperatur dispergiert. Sofern die Beschichtung weitere osteoinduktive oder biozide Materialien enthalten soll, werden diese ebenfalls in die Dispersion gegeben, in diesem Falle beträgt die Summe der Gewichte von PDLLA und weiteren Materialien 400 mg.

Die Dispersion wird für 6 h stehengelassen, bis eine kolloidale Lösung entstanden ist, die durch einen sterilen Mikrofilter mit einer Porengröße von 0,45 µm in ein steriles Gefäß filtriert wird.

Kirschner-Drähte (Durchmesser 1,6 mm, Länge 3,5 cm) aus Titan und Stahl sowie Titan-Knochennägel werden in die filtrierte Lösung eingetaucht, anschließend läßt man das Lösungsmittel während eines Zeitraums von 10 min in einer Chloroform-Atmosphäre abdampfen. Dieser Vorgang (Beschichten und Abdampfen) wird noch einmal wiederholt.

Die erhaltenen Implantate sind mit einer dünnen, lackartigen Polymerschicht überzogen, die Schichtdicke beträgt etwa 10 bis 20 µm.

### Beispiel 2: Mikrobiologische Eigenschaften der Beschichtung

Mikrobiologische Untersuchungen von Titan-Kirschner-Drähten mit einer erfindungsgemäßen PDLLA-Beschichtung zeigten nach einer 6-wöchigen und einer 12-wöchigen Inkubation kein feststellbares Wachstum von Mikroorganismen.

Ferner wurden je 10 erfindungsgemäß mit PDLLA beschichtete und 10 unbeschichtete Implantate mit Staphylokokken (KD 10⁶) kontaminiert. Bei den beschichteten Implantaten zeigte sich eine signifikant geringere Adhäsion dieser Mikroorganismen.

### Beispiel 3: Mechanische Stabilität der Beschichtung

Je 20 Kirschner-Drähte aus Titan und Stahl wurden gewogen und anschließend gemäß Bsp. 1 mit PDLLA beschichtet, das 1 % Methyiviolett als Farbindikator enthält.

Die Drähte wurden in die Tibiae von Ratten implantiert. Nach der Explantation wurde der mechanische Abrieb der Beschichtung durch Wiegen sowie photometrische Messungen festgestellt. Der höchste festgestellte Abrieb betrug 2,9 % bei Titandrähten und 4,6 % bei Stahidrähten. Eine rasterelektronenmikroskopische Untersuchung zeigte bei keinem der untersuchten Implantate einen Abrieb der Beschichtung bis auf die Metalloberfläche.

### Beispiel 4:

Dieses Beispiel zeigt die Vorteile einer kolloidalen Lösung für die mechanische Festigkeit der Beschichtung.

In 2 Ansätzen werden je 800 mg PDLLA R 203 in je 6 ml Ethylacetat gegeben. Die erhaltenen Dispersionen werden für eine Zeitdauer von 6 bzw. 24 h bei Raumtemperatur stehengelassen und wie in Bsp. 1 filtriert. Mit den erhaltenen Dispersionen bzw. Lösungen werden sogenannte Stents nach dem in Bsp. 1 angegebenen Verfahren beschichtet. Es sei angemerkt, daß es sich bei Stents nicht um Implantate im Sinne der Erfindung handelt. Sie werden lediglich deswegen genommen, weil sie sich besonders gut zur Durchführung von Dehnversuchen und damit Prüfung der mechanischen Festigkeit der lackartigen Beschichtung eignen.

Die Masse der Beschichtung wird durch Wiegen der Stents vor und nach dem Beschichten festgestellt.

Die beschichteten Stents werden in dem Fachmann geläufiger Weise mit einem PTCA-Ballon unter einem Überdruck von 8 bar aufgedehnt. Die aufgedehnten Stents werden wiederum gewogen und so der Verlust an Beschichtungsgewicht durch Abplatzen oder dergleichen bestimmt.

Man stellt fest, daß bei den Stents, die mit der vor dem Filtrieren 6 h stehengelassenen Dispersion beschichtet wurden, der durchschnittliche Beschichtungsverlust 0,8 % beträgt, bei den anderen Stents (24 h stehenlassen) 6,0 Gew.-%. Dies zeigt, daß für die mechanische Festigkeit der Beschichtung es vorteilhaft ist, keine vollständige chemische Lösung des Polymers im Lösungsmittel herzustellen, sondern eine kolloidale Lösung mit einer Kolloidteilchengröße von 0,45 µm oder kleiner.

### Beispiel 5: Stabilität der Wirksubstanzen in der Beschichtung

Um die Stabilität der eingearbeiteten Wachstumsfaktoren (WF) in die Beschichtung zu untersuchen, wurden Titan-Kirschner-Drähte gemäß Beispiel 1 mit PDLLA und eingearbeiteten Wachstumsfaktoren IGF-I (5 Gew.-%) und TGF-βI (lGew.-%) beschichtet. Die Stabilität (Lagerstabilität) der WF wurde nach 6 Wochen, nach 6 Monaten und nach 1 Jahr untersucht. Es fand sich ein Verlust von unter 3% Wirksamkeit innerhalb von 6 Wochen. Nach 6 Monaten konnten noch mehr als 95,5% und nach 1 Jahr mehr als 93% der in die Beschichtung eingearbeiteten WF wirksam nachgewiesen werden. Dies zeigt, daß die erfindungsgemäß in die Beschichtung eingearbeiteten Wirksubstanzen ihre biologische Stabilität und Wirksamkeit auch bei langfristiger Lagerung des beschichteten Implantats vor dessen Verwendung behalten.

### Beispiel 6: Biologisches Abbauverhalten der PDLLA-Beschichtung

Mit gemäß Bsp. 1 mit PDLLA beschichteten Titan -Kirschner-Drähten wurden Elutionsversuche in vitro vorgenommen. Um in vivo -Situationen zu simulieren, wurden die Elutionen in physiologischer 0,9% NaCl-Lösung bei einer Temperatur von 37°C unter laminar-air-flow-Bedingungen durchgeführt.

Binnen 9 Wochen wurde etwa 10% der PDLLA-Beschichtung kontinuierlich abgebaut.

Um das Abbauverhalten der PDLLA Beschichtung in vivo zu untersuchen, wurden 10 PDLLA beschichtete Kirschner-Drähte, mit definierter Beschichtungsmasse, in die Tibiae von Sprague-Dawley Ratten implantiert. Nach 6 Wochen wurden die Implantate entfernt und durch Bestimmung der Gewichtsdifferenz vor Implantation und nach Explantation sowie Messung der inhärenten Viskosität und des Molekulargewichtes der vollständig abgelösten Beschichtung der Abbau der PDLLA Beschichtung in vivo nach 6 Wochen ermittelt und mit den in vitro Daten verglichen.

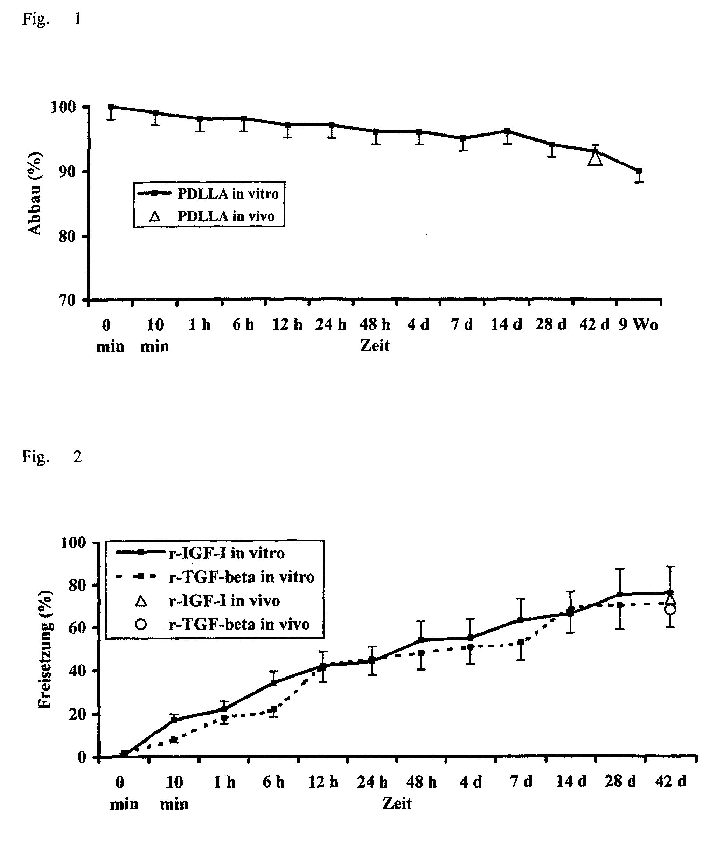

Das Ergebnis ist aus Fig. 1 ersichtlich. Binnen 9 Wochen wurde etwa 10 % der PDLLA-Beschichtung biologisch abgebaut. Die zum Vergleich durchgeführte in-vivo-Messung zeigt, daß sich in-vitro- und in-vivo-Ergebnisse zu diesem Zeitpunkt gut dekken.

### Beispiel 7: Untersuchung der Freisetzung von in die Beschichtung eingearbeiteten zusätzlichen Wirkstoffen

Gemäß Bsp. 1 wurden mit PDLLA beschichtete Titan-Kirschner-Drähte hergestellt, die Beschichtungen enthielten zusätzlich entweder 5 Gew.-% IGF-I oder 1 Gew.-% TGF-β1 oder 5 Gew.-% IGF-I und 1 Gew.-% TGF-β1 in Kombination.

Die Freisetzung dieser in die Beschichtung eingearbeiteten Wachstumsfaktoren wurde durch in-vitro-Elutionsversuche untersucht. Die Ergebnisse sind in Fig. 2 dargestellt. Binnen 48 h ergibt sich eine initiale Freisetzung von Wachstumsfaktoren aus der Beschichtung in der Größenordnung von 48 bis 54 %. Die weitere Freisetzung erfolgt kontinuierlich, bis nach 6 Wochen insgesamt zwischen 71 und 78 % der eingearbeiteten Wachstumsfaktoren freigesetzt worden sind.

Je 10 mit PDLLA und den genannten Wachstumsfaktoren beschichtete Titan-Kirschner-Drähte wurden in die Tibiae von Sprague Dawley Ratten implantiert. Die Implantate wurden nach 42 Tagen entfernt und die verbleibenden Konzentrationen der eingearbeiteten Wachstumsfaktoren mittels ELISA bestimmt. Aus Fig. 2 ist ersichtlich, daß sich die in-vivo-Ergebnisse mit den in-vitro-Elutionsversuchen decken.

### Beispiel 8: Osteoinduktiver Effekt der erfindungsgemäßen Implantate

In einem Tierexperiment wurden 60 Tiere (5 Monate alte weibliche Sprague Dawley Ratten) untersucht.

Alle Versuchstiere erhielten eine standardisierte Fraktur der rechten Tibia. Nach Reposition wurden in die Tibia unterschiedlich beschichtete Titandrähte (1,0 mm Durchmesser) als intramedullärer Kraftträger implantiert.

Postoperativ wurden bis zum 42. Tag je nach Gruppenzugehörigkeit (siehe unten) täglich 2 mg/kg ratten-spezifisches rekombinantes Wachstumshormon (r-rGH) bzw. Placebo subcutan injiziert. Zu den Zeitpunkten 0 d, 4 d, 7 d, 14 d, 21 d, 28 d, 35 d und 42 d wurden unter Inhalationsnarkose Röntgenaufnahmen in 2 Ebenen durchgeführt, jeweils 1,25 ml Blut retrobulbär entnommen (bei -80°C tiefgefroren), das Gewicht und die Körpertemperatur bestimmt. Am Tag 42 wurden die frakturierten und die unfrakturierten Tibiae unter Erhalt des Periost freipräpariert und anschließend biomechanisch getestet (torsionales Drehmoment - torsionale Steifigkeit).

### Gruppenaufteilung

- **Gruppe I:**: Fraktur der rechten Tibia - Implantat unbeschichtet -
Systemische Applikation von Placebo (Kontrollgruppe)
- **Gruppe II:**: Fraktur der rechten Tibia - Implantat beschich tet mit Poly-D,L-laktid (R 203) -
Systemische Applikation von Placebo
- **Gruppe III:**: Fraktur der rechten Tibia - Implantat beschichtet mit Poly-D,L-laktid -
Systemische Applikation von (r-rGH)
- **Gruppe IV:**: Fraktur der rechten Tibia - Implantat beschichtet mit Poly-D,L-laktid und Wachstumsfaktoren IGF-I (5 %) und TGF-β (1 %)
Systemische Applikation von Placebo
- **Gruppe v:**: Fraktur der rechten Tibia - Implantat beschichtet mit Poly-D,L-laktid und Wachstumsfaktoren IGF-I (5 %) und TGF-β (1 %)
Systemische Applikation von (r-rGH)

Die beschichteten Implantate wurden gemäß Bsp. 1 hergestellt.

### Ergebnisse:

### Fraktursetzung

Das Frakturmodell erwies sich als gut geeignet, eine standardisierte Querfraktur der rechten Tibia ohne großen Weichteilschaden zu erzeugen. Bei 2 von 60 war eine Trümmerfraktur und eine Spiralfraktur der Tibia zu verzeichnen, die zur vorzeitigen Beendigung führten. Ein Tier verstarb bei einer Nachuntersuchung in Narkose (32. Tag).

### Gewicht und Temperatur

Bei den systemisch mit (r-rGH) behandelten Tieren (Gruppe III und V) kam es im Vergleich zu den Placebo verabreichten Tieren (Gruppe I, II und IV) zu keinem Anstieg der Körpertemperatur im Verlauf, jedoch zu einer signifikanten Erhöhung des Körpergewichtes um 13 % (p<0,05). Signifikante Unterschiede in den Gruppen I, II, und IV (Placebo) bzw. III und V (GH) waren nicht zu verzeichnen.

### Biomechanische Testung

Die vorliegenden Daten wurden nach absoluten Werten (torsionales Drehmoment) und prozentual (torsionale Steifigkeit) im Vergleich zur nicht frakturierten Gegenseite erfaßt.

Die Ergebnisse verdeutlichen eine signifikante (p<0,05) Zunahme des maximalen Drehmomentes der Gruppe III und der Gruppen IV und V gegenüber der systemischen Applikation. Die lokale Applikation von Wachstumsfaktoren (Gruppe IV) scheint hierbei nicht nur deutlich das maximale Drehmoment gegenüber der Kontrollgruppe zu erhöhen, es werden im Mittel auch höhere Ergebnisse gegenüber der systematischen Applikation von r-rGH beobachtet (nicht signifikant). Eine weitere Steigerung des maximalen Drehmoments durch gleichzeitige Gabe von r-rGH und lokale Applikation von IGF-I und TGF-β kann nicht beobachtet werden. Des weiteren konnte eine signifikante Steigerung des maximalen Drehmoments der mit Poly-D,L-laktid behandelten Gruppe gegenüber der Kontrollgruppe I festgestellt werden.

Bei der torsionalen Steifigkeit im Vergleich zur kontralateralen Tibia zeigt sich ein vergleichbares Bild. Auch hier zeigen die Gruppen mit der lokalen Applikation von Wachstumsfaktoren die günstigsten Ergebnisse.

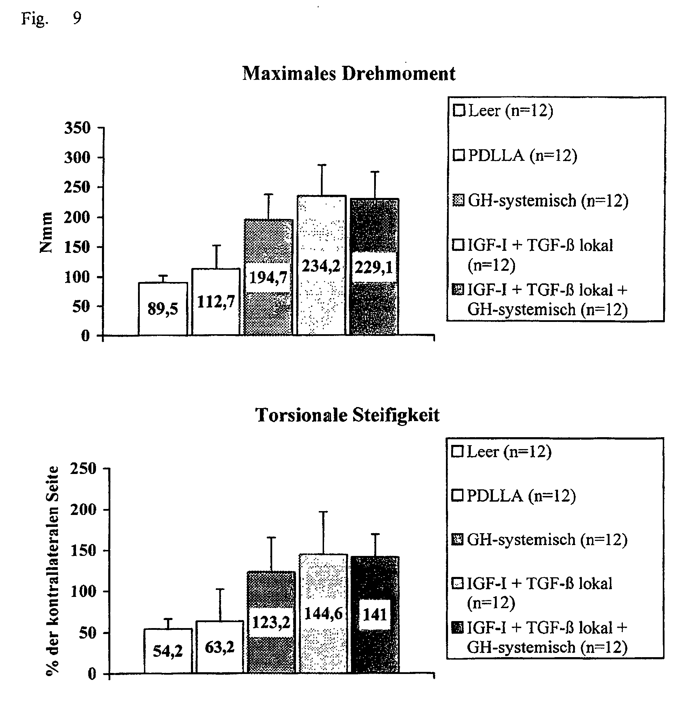

Die Ergebnisse sind in Fig. 9 zusammengefaßt.

### Beispiel 9:

Bei 5 Monate alten weiblichen Sprague Dawley Ratten (n=144) wurde eine standardisierte geschlossene Fraktur der rechten Tibia mit einer Frakturmaschine erzeugt und mit unbeschichteten versus beschichteten Titan-Kirschner-Drähten intramedullär stabilisiert. Hierbei wurden folgende Gruppen miteinander verglichen:
- **Gruppe I:**: Implantat unbeschichtet (Kontrollgruppe)
- **Gruppe II:**: Implantat beschichtet mit PDLLA (R 203)
- **Gruppe III:**: Implantat beschichtet mit PDLLA + r-IGF-I (5 %)
- **Gruppe IV:**: Implantat beschichtet mit PDLLA + r-IGF-I (5%)+ TGF-β1 (1 %)

Die beschichteten Implantate wurden gemäß Bsp. 1 hergestellt.

Es erfolgten Röntgenaufnahmen in 2 Ebenen (a.-p. und lateral) im zeitlichen Verlauf. Zu den Zeitpunkten 0 d, 4 d, 7 d, 14 d, 21 d, 28 d wurden Serumwerte, einschließlich der systemischen Konzentration von r-IGF-I und r-TGF-β1, das Körpergewicht und die Körpertemperatur bestimmt. Nach 4 Wochen wurden die Implantate entfernt und die frakturierten Tibiae im Vergleich zur unbehandelten Gegenseite beiomechanisch getestet. Die histomorphometrischen Untersuchung (Safranin O./v. Kossa) der Kalli wurden mit einem Bildanalysesystem (Zeiss KS 400) quantifiziert.

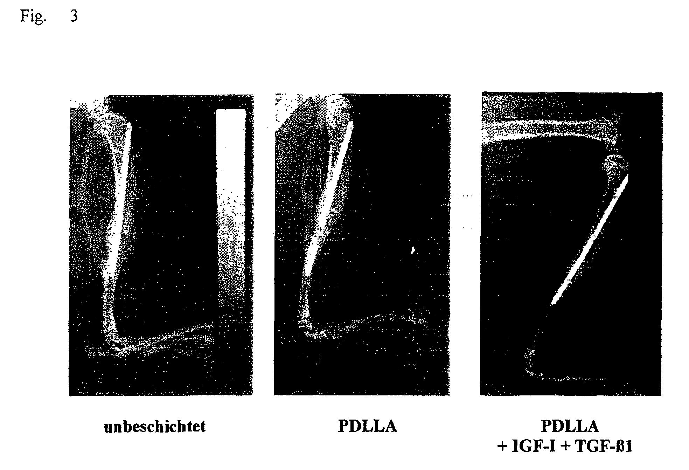

In der radiologischen Auswertung war bei der unbehandelten Gruppe I noch deutlich ein Frakturspalt zu erkennen. In den Gruppen II und III zeigte sich eine gute Kallusformation im Vergleich zu der unbeschichteten Gruppe I. Die Tiere der Gruppe IV zeigten nahezu vollständig konsolidierte Frakturen (Fig. 3).

Die biomechanischen Untersuchungen ergaben im Vergleich zur unbehandelten Gegenseite ein signifikant höheres maximales Drehmoment und maximale torsionale Steifigkeit der Gruppe IV im Vergleich zu allen anderen Gruppen. Die kombinierte Applikation von r-IGF-I und r-TGF-β1 ergab hierbei ein signifikant höheres maximales Drehmoment und maximale torsionale Steifigkeit im Vergleich zu der mit IGF-I behandelten Gruppe.

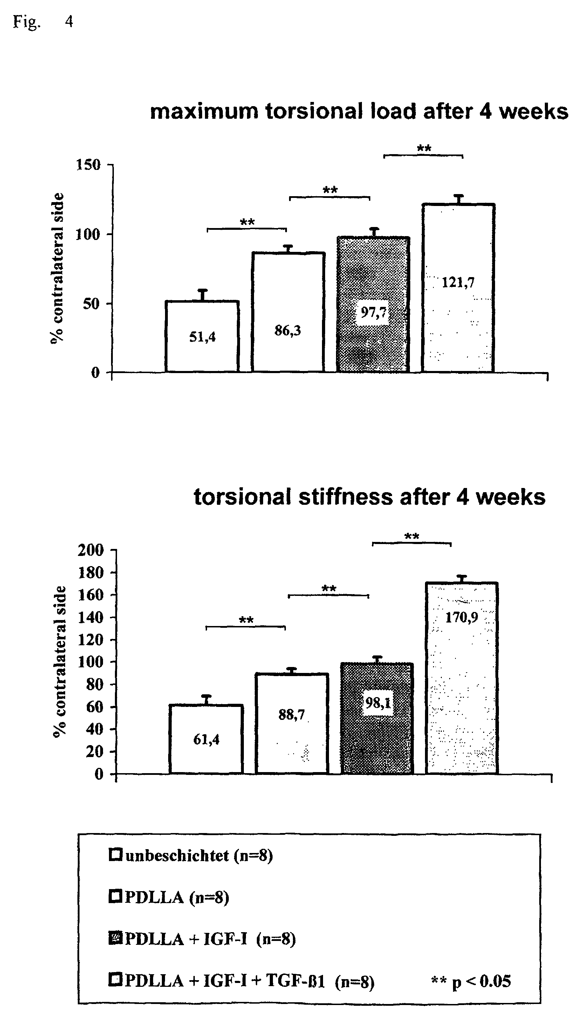

Die Polylaktid-behandelte Gruppe zeigte signifikant höheres maximales Drehmoment und maximale torsionale Steifigkeit im Vergleich zur unbehandelten Gruppe I (Fig. 4).

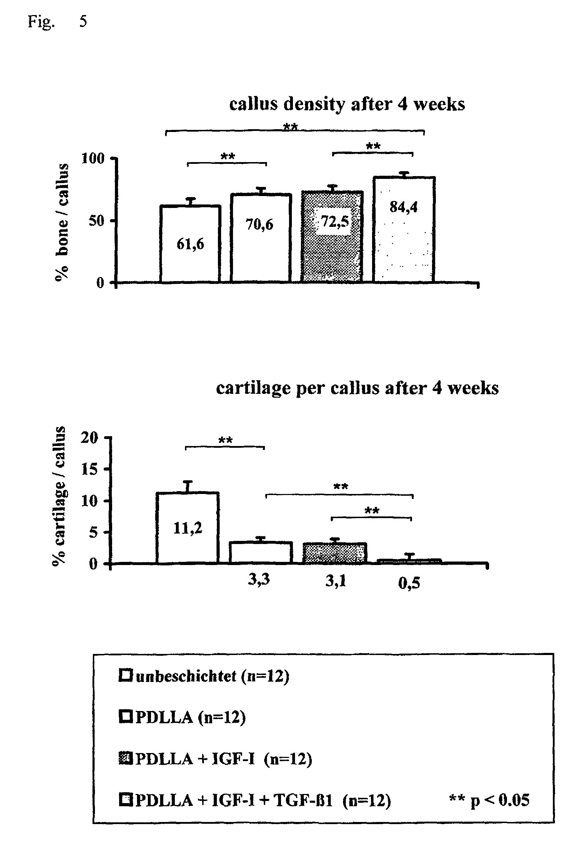

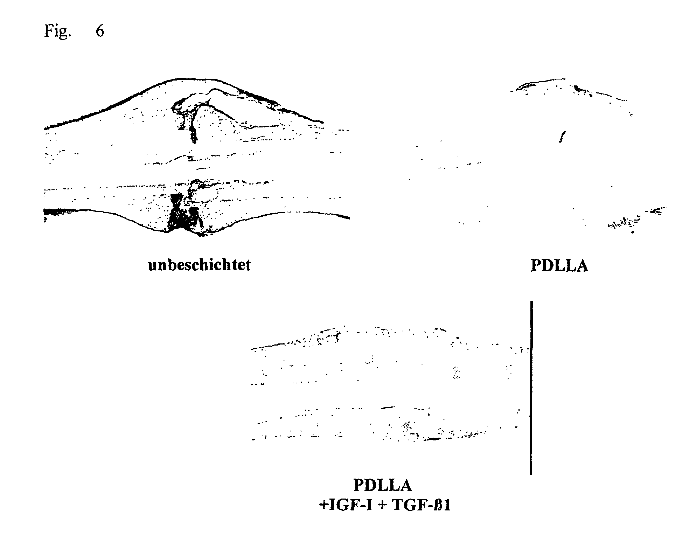

Die histomorphometrischen Untersuchungen bestätigen die radiologischen und biomechanischen Ergebnisse. Es zeigten sich signifikant mehr Bereiche mit Bindegewebszellen in der Gruppe I im Vergleich zu den behandelten Gruppen. Die mit PDLLA behandelte Gruppe zeigte eine gute Kallusformation und ein Bild von fortgeschrittenem Kallusremodelling mit geringen Anteilen von Bindegewebszellen. Gruppe IV zeigte das Bild einer nahezu vollständig remodelten Fraktur und die höchste Knochendichte im Kallus. Die nur mit Polylaktid behandelte Gruppe zeigte ebenfalls eine signifikant höhere Knochendichte im Kallusbereich verglichen mit der Kontrollgruppe (Fig. 5 und 6).

Es zeigten sich keine Veränderungen der Serumparameter, des Körpergewichts und der Körpertemperatur der behandelten im Vergleich zu den unbehandelten Gruppen.

### Beispiel 10:

Bei 12 Monate alten Yukatan-Minischweinen (n=30) wurde eine standardisierte Osteotomie (1 mm Spalt) der rechten Tibia durchgeführt und mit beschichteten als auch unbeschichteten Titan-Tibia-Nägeln intramedullär stabilisiert und statisch verriegelt. Folgende Gruppen wurden miteinander verglichen:
- **Gruppe I:**: Implantat unbeschichtet (Kontrollgruppe)
- **Gruppe II:**: Implantat beschichtet mit PDLLA (R 203)
- **Gruppe III:**: Implantat beschichtet mit PDLLA + r-IGF-I (5%) + TGF-β1 (1 %)

Die beschichteten Implantate wurden gemäß Bsp. 1 hergestellt.

Es folgten radiologische Untersuchungen sowie Serumuntersuchungen im Verlauf. Nach 4 Wochen wurden die beiden Tibiae entnommen und biomechanisch getestet. Der Kallusdurchmesser wurde gemessen sowie das Kallusvolumen nach dem archimedischem Prinzip bestimmt.

### Ergebnisse:

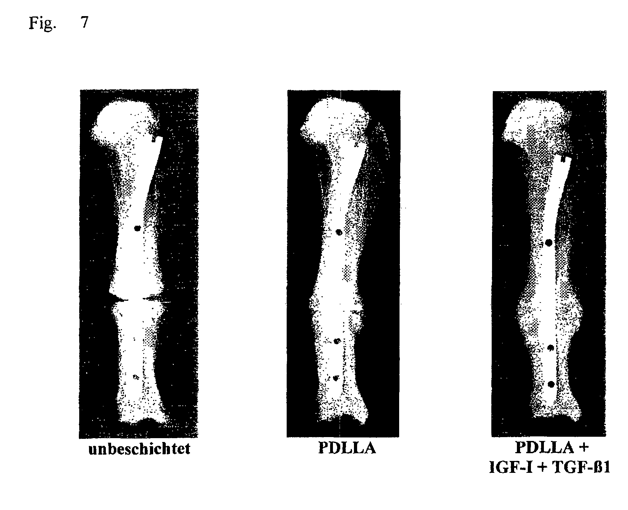

Nach 4 Wochen zeigten alle Tiere der Kontrollgruppe eine inkomplette Konsolidierung des Osteotomiespaltes. Die mit Polylaktid behandelte Gruppe zeigte eine gute Kallusformation. In der Gruppe III zeigte sich eine weit fortgeschrittene Kallusbildung (Fig. 7).

Das Kallusvolumen und der Kallusdurchmesser war in der mit Polylaktid behandelten Gruppe II und der mit zusätzlichen Wachstumsfaktoren behandelten Gruppe III signifikant größer im Vergleich zur Kontrollgruppe.

Im Vergleich zur Gegenseite zeigte sich in der mit Polylaktid behandelten Gruppe ein signifikant höheres maximales torsionales Drehmoment und maximale torsionale Steifigkeit im Vergleich zur Kontrollgruppe.

Durch Einarbeiten von Wachstumsfaktoren in die Polylaktidbeschichtung konnten eine signifikante Zunahme des maximalen torsionalen Drehmoments und der maximalen torsionalen Steifigkeit erreicht werden.

### Festigkeit der intramedullären Kraftträger

Die standardisierte Explantation der Titandrähte aus den Tibiae mit einem Kraftaufnehmer ergab eine signifikant höher aufzubringende Kraft bei der Explantation der mit IGF-I und TGF-β beschichteten Drähte gegenüber der Kontrollgruppe.

Die Beispiele 8 bis 10 zeigen, daß sich mittels eines erfindungsgemäß beschichteten Implantats die Osteosynthese und damit die Frakturheilung signifikant beschleunigen läßt. Diese Beschleunigung ist nachweisbar für ein polymerbeschichtetes Implantat ohne Zusatz weiterer osteoinduktiver Wirkstoffe. Durch die Einarbeitung von Wachstumsfaktoren in die Beschichtung läßt sich die Frakturheilung weiter beschleunigen, wobei die kombinierte Applikation von IGF-I und TGF-β besonders vorteilhaft ist.

Die Beispiele zeigen ferner, daß sich durch das erfindungsgemäße Verfahren eine lackartige Beschichtung herstellen läßt, die sich durch ihre physikalische Struktur und mechanische Festigkeit von jeglichem Stand der Technik deutlich unterscheidet.

## Patentansprüche

1. Implantat zur Versorgung von pathologischen Veränderungen am Stütz- und/oder Bewegungsapparat, **dadurch gekennzeichnet, daß** sie eine lackartige Beschichtung einer Stärke von 100 µm oder weniger aus einem biologisch abbaubaren Polymer aufweist, wobei die lackartige Beschichtung erhältlich ist durch ein Verfahren mit den Schritten:
- Herstellen einer Dispersion des biologisch abbaubaren Polymers in einem organischen Lösungsmittel,
- Aufbringen der Dispersion auf die zu beschichtende Oberfläche,
- Abdampfenlassen des Lösungsmittels in einer mit Lösungsmitteldampf im wesentlichen gesättigten Gasatmosphäre.

2. Implantat nach Anspruch 1, **dadurch gekennzeichnet, daß** es ausgewählt ist aus der Gruppe bestehend aus Frakturfixationsvorrichtungen und Endoprothesen.

3. Implantat nach Anspruch 2, **dadurch gekennzeichnet, daß** die Frakturfixationsvorrichtung ausgewählt ist aus der Gruppe bestehend aus Platten, Schrauben, Nägeln, Stiften, Drähten, Fäden oder Cages für den Stütz- und Bewegungsapparat.

4. Implantat nach einem der Ansprüche 1 bis 3, **dadurch gekennzeichnet, daß** die lackartige Beschichtung eine Stärke von 50 µm oder weniger, vorzugsweise 30 µm oder weniger, weiter vorzugsweise 20 µm oder weniger aufweist.

5. Implantat nach Anspruch 4, **dadurch gekennzeichnet, daß** die lackartige Beschichtung eine Stärke von 10 bis 30 µm, vorzugsweise 10 bis 20 µm aufweist.

6. Implantat nach einem der Ansprüche 1 bis 5, **dadurch gekennzeichnet, daß** das Polymer eine Glasübergangstemperatur von 37°C oder höher besitzt.

7. Implantat nach einem der Ansprüche 1 bis 6, **dadurch gekennzeichnet, daß** das Polymer ein mittleres Molekulargewicht von 100 kDa oder weniger besitzt.

8. Implantat nach einem der Ansprüche 1 bis 7, **dadurch gekennzeichnet, daß** das Polymer ausgewählt ist aus der Gruppe bestehend aus Poly-α-Hydroxysäuren, Polyglykolen, Polytyrosincarbonaten, Stärke, Gelatine und Cellulose sowie deren Mischungen und Mischpolymerisaten daraus.

9. Implantat nach Anspruch 8, **dadurch gekennzeichnet, daß** das Polymer Poly-α-Hydroxysäuren ausgewählt aus der Gruppe bestehend aus Polylaktiden, Polyglykolsäuren und Mischpolymerisaten daraus umfaßt.

10. Implantat nach einem der Ansprüche 1 bis 7, **dadurch gekennzeichnet, daß** die lackartige Beschichtung zusätzliche pharmazeutisch wirksame Stoffe enthält.

11. Implantat nach Anspruch 10, **dadurch gekennzeichnet, daß** die pharmazeutisch wirksamen Stoffe osteoinduktive Materialien umfassen.

12. Implantat nach Anspruch 11, **dadurch gekennzeichnet, daß** das osteoinduktive Material einen oder mehrere Wachstumsfaktoren enthält.

13. Implantat nach Anspruch 12, **dadurch gekennzeichnet, daß** der Anteil der Wachstumsfaktoren am Gesamtgewicht der Beschichtung 0,1 - 10 Gew.-%, vorzugsweise 0,5 - 8 Gew.-%, weiter vorzugsweise 1 - 5 Gew.-% beträgt.

14. Implantat nach Anspruch 12, **dadurch gekennzeichnet, daß** die Wachstumsfaktoren ausgewählt sind bestehend aus der Gruppe der IGF, TGF, FGF, EGF, BMP sowie der PDGF.

15. Implantat nach Anspruch 12, **dadurch gekennzeichnet, daß** die lackartige Beschichtung IGF-I und/oder TGF-β enthält.

16. Implantat nach Anspruch 12, **dadurch gekennzeichnet, daß** die lackartige Bescbichtung eine Kombination von IGF-I und TGF-β enthält.

17. Verfahren zur Herstellung eines Implantat nach einem der Ansprüche 1 bis 16, mit den Schritten:
- Herstellen einer Dispersion des biologisch abbaubaren Polymers in einem organischen Lösungsmittel,
- Aufbringen der Dispersion auf die zu beschichtende Oberfläche,
- Abdampfenlassen des Lösungsmittels in einer mit Lösungsmitteldampf im wesentlichen gesättigten Gasatmosphäre.

18. Verfahren nach Anspruch 17, **dadurch gekennzeichnet, daß** das Aufbringen und Abdampfenlassen bei einer Temperatur von 0 - 30°C, vorzugsweise etwa 22°C erfolgt.

19. Verfahren nach einem der Anspruche 17 bis 18, **dadurch gekennzeichnet, daß** das Aufbringen der Dispersion, und Abdampfenlassen des Lösungsmittels zweimal oder mehrmals wiederholt wird.

20. Verfahren nach einem der Ansprüche 17 bis 19, **dadurch gekennzeichnet, daß** die Dispersion eine kolloidale Lösung des Polymers im Lösungsmittel ist.

21. Verfahren nach Anspruch 20, **dadurch gekennzeichnet, daß** die kolloidale Lösung hergestellt wird durch Stehenlassen eines Gemischs von Polymer und Lösungsmittel für einen Zeitraum von 1 min - 24 h, vorzugsweise 2 - 24 h, weiter vorzugsweise 3 - 12 h, weiter vorzugsweise 4 - 8 h, weiter vorzugsweise etwa 6 h.

22. Verfahren nach Anspruch 20 oder 21, **dadurch gekennzeichnet, daß** die kolloidale Lösung vor dem Aufbringen filtriert wird.

23. Verfahren nach Anspruch 22, **dadurch gekennzeichnet, daß** die kolloidale Lösung durch einen Mikroporenfilter mit einer Porengröße von 0,45 µm oder kleiner filtriert wird.

24. Verfahren nach einem der Ansprüche 17 bis 23, dadurch gekeimzeichnet, daß als Lösungsmittel Ethylacetat oder Chloroform verwendet wird.

25. Verfahren nach einem der Ansprüche 17 bis 24, **dadurch gekennzeichnet, daß** die Dispersion 20 - 300, vorzugsweise 50 - 150 mg Polymer pro ml Lösungsmittel enthält.

26. Implantat nach einem der Ansprüche 1 bis 16, erhältlich durch ein Verfahren nach einem der Ansprüche 17 bis 25.

## Claims

1. Implant for treating pathological changes of the support and/or locomotor apparatus, **characterized in that** it has a paint-like coating with a thickness of 100 µm or less made of a biodegradable polymer, the paint-like coating being obtainable by a method with the steps of:
- producing a dispersion of the biodegradable polymer in an organic solvent,
- applying the dispersion onto the surface to be coated,
- allowing the solvent to evaporate off in a gas atmosphere substantially saturated with solvent vapour.

2. Implant according to Claim 1, **characterized in that** it is chosen from the group including fracture fixation devices and endoprostheses.

3. Implant according to Claim 2, **characterized in that** the fracture fixation device is chosen from the group including plates, screws, nails, pins, wires, filaments or cages for the support and locomotor apparatus.

4. Implant according to one of Claims 1 to 3, **characterized in that** the paint-like coating has a thickness of 50 µm or less, preferably 30 µm or less, more preferably 20 µm or less.

5. Implant according to Claim 4, **characterized in that** the paint-like coating has a thickness of 10 to 30 µm, preferably 10 to 20 µm.

6. Implant according to one of Claims 1 to 5, **characterized in that** the polymer has a glass transition temperature of 37°C or higher.

7. Implant according to one of Claims 1 to 6, **characterized in that** the polymer has a mean molecular weight of 100 kDa or less.

8. Implant according to one of Claims 1 to 7, **characterized in that** the polymer is chosen from the group including poly-α-hydroxy acids, polyglycols, polytyrosine carbonates, starch, gelatin and cellulose, and also mixtures and copolymers thereof.

9. Implant according to Claim 8, **characterized in that** the polymer poly-α-hydroxy acids is chosen from the group including polylactides, polyglycolic acids and copolymers thereof.

10. Implant according to one of Claims 1 to 7, **characterized in that** the paint-like coating contains additional pharmaceutically active substances.

11. Implant according to Claim 10, **characterized in that** the pharmaceutically active substances include osteoinductive materials.

12. Implant according to Claim 11, **characterized in that** the osteoinductive material contains one or more growth factors.

13. Implant according to Claim 12, **characterized in that** the proportion of growth factors to the total weight of the coating is 0.1 - 10% by weight, preferably 0.5 - 8% by weight, more preferably 1 - 5% by weight.

14. Implant according to Claim 12, **characterized in that** the growth factors are chosen from the group of IGF, TGF, FGF, EGF, BMP and PDGF.

15. Implant according to Claim 12, **characterized in that** the paint-like coating contains IGF-I and/or TGF-β.

16. Implant according to Claim 12, **characterized in that** the paint-like coating contains a combination of IGF-I and TGF-β.

17. Method for producing an implant according to one of Claims 1 to 16, with the steps of:
- producing a dispersion of the biodegradable polymer in an organic solvent,
- applying the dispersion onto the surface to be coated,
- allowing the solvent to evaporate off in a gas atmosphere substantially saturated with solvent vapour.

18. Method according to Claim 17, **characterized in that** the application and evaporation takes place at a temperature of 0 - 30°C, preferably approximately 22°C.

19. Method according to one of Claims 17 to 18, **characterized in that** the application of the dispersion and evaporation of the solvent is repeated twice or more.

20. Method according to one of Claims 17 to 19, **characterized in that** the dispersion is a colloidal solution of the polymer in the solvent.

21. Method according to Claim 20, **characterized in that** the colloidal solution is produced by leaving a mixture of polymer and solvent to stand for a period of 1 minute to 24 hours, preferably 2 to 24 hours, more preferably 3 to 12 hours, more preferably 4 to 8 hours, more preferably approximately 6 hours.

22. Method according to Claim 20 or 21, **characterized in that** the colloidal solution is filtered before application.

23. Method according to Claim 22, **characterized in that** the colloidal solution is filtered through a microporous filter with a pore size of 0.45 µm or smaller.

24. Method according to one of Claims 17 to 23, **characterized in that** ethyl acetate or chloroform is used as solvent.

25. Method according to one of Claims 17 to 24, **characterized in that** the dispersion contains 20 - 300, preferably 50 - 150 mg of polymer per ml of solvent.

26. Implant according to one of Claims 1 to 16, obtainable by a method according to one of Claims 17 to 25.

## Revendications

1. Implant pour soigner des modifications pathologiques du système protecteur et/ou moteur, **caractérisé en ce qu'**il présente un revêtement de type laque d'une épaisseur de 100 µm ou moins en un polymère biologiquement dégradable, où le revêtement de type laque peut être obtenu par un procédé comprenant les étapes consistant en :
- préparer une dispersion du polymère biologiquement dégradable dans un solvant organique ;
- appliquer la dispersion sur la surface à revêtir, et
- laisser s'évaporer le solvant dans une atmosphère gazeuse essentiellement saturée de la vapeur du solvant.

2. Implant selon la revendication 1, **caractérisé en ce qu'**il est choisi parmi le groupe consistant en les dispositifs fixateurs pour fracture et les endoprothèses.

3. Implant selon la revendication 2, **caractérisé en ce que** le dispositif fixateur pour fractures est choisi parmi le groupe consistant en les plaques, les vis, les clous, les pointes, les treillis, les fils ou les cages pour le système protecteur et/ou moteur.

4. Implant selon l'une quelconque des revendications 1 à 3, **caractérisé en ce que** le revêtement de type laque présente une épaisseur de 50 µm ou moins, de préférence de 30 µm ou moins, de manière encore préférée de 20 µm ou moins.

5. Implant selon la revendication 4, **caractérisé en ce que** le revêtement de type laque présente une épaisseur allant de 10 à 30 µm, de préférence de 10 à 20 µm.

6. Implant selon l'une quelconque des revendications 1 à 5, **caractérisé en ce que** le polymère possède une température de transition vitreuse de 37°C ou supérieure.

7. Implant selon l'une quelconque des revendications 1 à 6, **caractérisé en ce que** le polymère possède un poids moléculaire moyen de 100 kDa ou moins.

8. Implant selon l'une quelconque des revendications 1 à 7, **caractérisé en ce que** le polymère est choisi parmi le groupe consistant en les poly(α-hydroxyacides), les polyglycols, les polytyrosinecarbonates, les amidons, les gélatines et les celluloses ainsi que leurs mélanges et copolymères.

9. Implant selon la revendication 8, **caractérisé en ce que** le polymère poly(α-hydroxyacide) est choisi parmi le groupe consistant en les polylactides, les poly(acides glycoliques) et les copolymères de ceux-ci.

10. Implant selon l'une quelconque des revendications 1 à 7, **caractérisé en ce que** le revêtement de type laque contient en outre, des substances pharmaceutiquement actives.

11. Implant selon la revendication 10, **caractérisé en ce que** les substances pharmaceutiquement actives comprennent les matériaux ostéoinductifs.

12. Implant selon la revendication 11, **caractérisé en ce que** le matériau ostéoinductif contient un ou plusieurs facteurs de croissance.

13. Implant selon la revendication 12, **caractérisé en ce que** la quantité de facteur de croissance se situe dans l'intervalle allant de 0,1 à 10% en poids, de préférence de 0,5 à 8% en poids, de manière encore préférée de 1 à 5% en poids, sur le poids total du revêtement.

14. Implant selon la revendication 12, **caractérisé en ce que** les facteurs de croissance sont choisis parmi le groupe consistant en les IGF, TGF, FGF, EGF, BMP, ainsi que PDGF.

15. Implant selon la revendication 12, **caractérisé en ce que** le revêtement de type laque contient l'IGF-I et/ou le TGF-β.

16. Implant selon la revendication 12, **caractérisé en ce que** le revêtement de type laque contient une combinaison d'IGF-I et de TGF-β.

17. Procédé de préparation d'un implant d'après l'une quelconque des revendications 1 à 16, comprenant les étapes consistant à :
- préparer une dispersion du polymère biologiquement dégradable dans un solvant organique ;
- appliquer la dispersion sur la surface à revêtir, et
- laisser s'évaporer le solvant dans une atmosphère gazeuse essentiellement saturée de la vapeur du solvant.

18. Procédé selon la revendication 17, **caractérisé en ce que** l'application et l'évaporation sont réalisées à une température allant de 0 à 30°C, de préférence d'environ 22°C.

19. Procédé selon l'une quelconque des revendications 17 à 18, **caractérisé en ce que** l'application de la dispersion et l'évaporation du solvant sont répétés deux ou plusieurs fois.

20. Procédé selon l'une quelconque des revendications 17 à 19, **caractérisé en ce que** la dispersion est une solution colloïdale du polymère dans le solvant.

21. Procédé selon la revendication 20, **caractérisé en ce que** la solution colloïdale est préparée par repos d'un mélange du polymère et du solvant pendant une période allant de 1 minute à 24 heures, de préférence de 2 à 24 heures, de manière encore préférée de 3 à 12 heures, de manière encore plus préférée de 4 à 8 heures, en particulier environ 6 heures.

22. Procédé selon la revendication 20 ou 21, **caractérisé en ce que** la solution colloïdale est filtrée avant l'application.

23. Procédé selon la revendication 22, **caractérisé en ce que** la solution colloïdale est filtrée sur un filtre microporeux avec une taille des pores de 0,45 µm ou moins.

24. Procédé selon l'une quelconque des revendications 17 à 23, **caractérisé en ce que** l'on utilise comme solvant, l'acétate d'éthyle ou le chloroforme.

25. Procédé selon l'une quelconque des revendications 17 à 24, **caractérisé en ce que** la dispersion contient 20 à 300, de préférence 50 à 150 mg de polymère par ml de solvant.

26. Implant selon l'une quelconque des revendications 1 à 16, pouvant être obtenu par un procédé selon l'une quelconque des revendications 17 à 25.
